(19) Europäisches Patentamt

European Patent Office

Office européen des brevets

(11) **EP 0 991 386 B1**

(12) **EUROPÄISCHE PATENTSCHRIFT**

| | |
|---|---|
| (45) Veröffentlichungstag und Bekanntmachung des Hinweises auf die Patenterteilung:<br>**17.10.2001 Patentblatt 2001/42** | (51) Int Cl.$^7$: **A61F 13/02** |
| (21) Anmeldenummer: **98934950.1** | (86) Internationale Anmeldenummer:<br>**PCT/EP98/03524** |
| (22) Anmeldetag: **10.06.1998** | (87) Internationale Veröffentlichungsnummer:<br>**WO 99/00080 (07.01.1999 Gazette 1999/01)** |

(54) **PFLASTER**

PLASTER

EMPLATRE

| | |
|---|---|
| (84) Benannte Vertragsstaaten:<br>**AT BE CH CY DE DK ES FI FR GB GR IE IT LI LU MC NL PT SE** | (72) Erfinder: **MALOWANIEC, Krzysztof, D.**<br>**D-89522 Heidenheim (DE)** |
| (30) Priorität: **25.06.1997 DE 19727032** | (74) Vertreter: **Friz, Oliver et al**<br>**Patentanwälte,**<br>**Dreiss, Fuhlendorf, Steimle & Becker,**<br>**Postfach 10 37 62**<br>**70032 Stuttgart (DE)** |
| (43) Veröffentlichungstag der Anmeldung:<br>**12.04.2000 Patentblatt 2000/15** | |
| (73) Patentinhaber: **Paul Hartmann Aktiengesellschaft**<br>**89522 Heidenheim (DE)** | (56) Entgegenhaltungen:<br>**EP-A- 0 353 972    WO-A-93/07843**<br>**US-A- 3 885 559    US-A- 5 088 483**<br>**US-A- 5 153 040** |

## Beschreibung

Die Erfindung betrifft ein Pflaster mit den Merkmalen des Oberbegriffs des Anspruchs 1 und einen Pflasterstreifen mit den Merkmalen des Oberbegriffs des Anspruchs 2 sowie ein Inzisionspflaster mit den Mekmalen des Oberbegriffs des Anspruchs 3. Es kann sich also um ein Pflaster, aber auch um einen Pflasterstrip, einen postoperativen Wundverband, ein Fixierpflaster oder eine Inzisionsfolie handeln. In den beiden letztgenannten Fällen ist in der Regel ein durchgehender haftender Bereich (Klebefläche) vorgesehen.

Aus EP-A-0 353 972 ist ein Pflaster mit einer Wundauflage und unterschiedlich stark haftenden Bereichen bekannt.

Bei einem Pflaster besteht ein Problem darin, dass es zwar einerseits gut auf der eine Wunde umgebenden Haut haften soll; es soll aber andererseits so sanft haften und wieder lösbar sein, dass die vorgeschädigte Haut geschont wird. Es soll auch möglichst schmerzfrei abziehbar sein.

Erfindungsgemäß wird diese Aufgabe durch die Merkmale der Ansprüche 1, 2 bzw. 3 gelöst. Vorteilhafte Weiterbildungen der Erfindung ergeben sich aus den Unteransprüchen.

Es wird also der äußere haftende Bereich, also die außen angebrachte zweite Klebezone, mit stärkerer Haftkraft ausgebildet. Diese Klebezone ist bei einem Pflaster von der Wunde weiter entfernt als die erste Klebezone, die geringere Haftkrafc aufweist. Man kann also in der zweiten Klebezone eine Schicht mit großer Haftkraft auftragen, die für die erwünschte gute und dauerhafte Haftung des Pflasters auf der eine Wunde umgebenden Haut des Patienten ausreicht. Die innere erste Klebezone kann dann vergleichsweise gering haftend ausgebildet sein. Sie muss im Grund nur dauerhaft die Lage des Pflasters, und damit z.B. die Umgebung eines Wundkissens sichern. Auf diese Weise kann man trotz einer insgesamt großen Haftzone sicherstellen, dass beim Abziehen die vorgeschädigte Haut nicht mehr als unbedingt nötig, d.h. sehr viel weniger als seither, belastet wird.

In den drei Figuren sind drei Ausführungsbeispiele der Erfindung beschrieben.

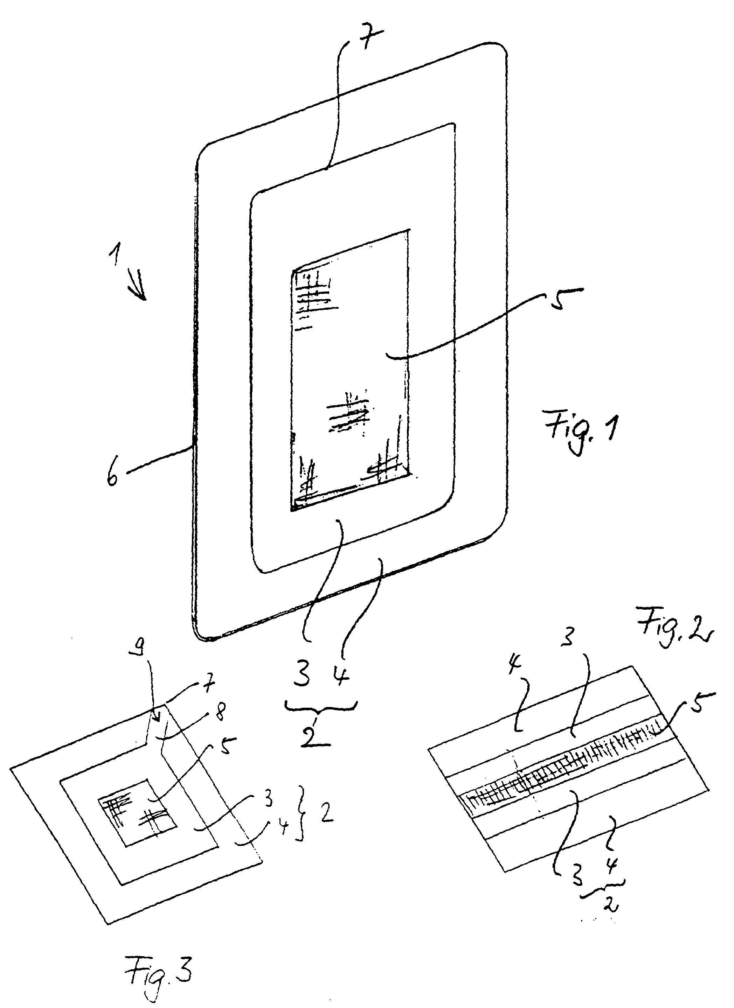

Figur 1 zeigt ein Pflaster 1, das einen haftenden Bereich 2 aufweist, der in eine erste innere Klebezone 3 und eine zweite äußere Klebezone 4 aufgeteilt ist. Die innere Klebezone 3 umgibt unmittelbar ein Wundkissen 5; die äußere Klebezone 4 umgibt unmittelbar die Klebezone 3. Die Grenze ist mit 7 bezeichnet. Der dem Betrachter der beigefügten Zaichnung zugewandte Bereich ist dann üblicherweise noch durch eine (nicht dargestellte) Schutzfolie abgedeckt, die vor dem Aufbringen des Pflasters abgezogen wird.

Die Haftkraft der inneren Klebezone 3 ist relativ schwach und liegt im Bereich von 0,5 bis 2 N/25cm. Das bedeutet, dass zum Ablösen eines Streifens von 25 mm Breite eine Kraft dieser Größe erforderlich ist (vgl. die Beschreibung der Prüfmethode am Ende der vorliegenden Beschreibung). Die Haftkraft der äußeren zweiten Klebezone 4 ist stärker und liegt im Bereich von 2 - 8 N/25 mm.

Die Unterlage 6, auf der die beiden Klebezonen 3,4 und das Wundkissen angebracht sind, kann aus einem textilen Flächengewebe wie z.B. einem Gewebe oder einem Gestrick oder einem Gewirk bestehen oder aber aus einem Vlies oder einer Folie, die in üblicher Weise mit Haftkleber, vorzugsweise durchgehend beschichtet sind. Es sind dabei ein- und mehrschichtige, auch heterogene Strukturen einsetzbar.

Als Anwendung der Erfindung kommen nicht nur Pflaster der in der Zeichnung gezeigten Art, sondern auch Pflasterstrips, postoperative Wundverbände und Fixierpflaster für Nadeln, Kanülen oder Verbände in Frage. Bei der Verwendung als Fixierpflaster für eine Kanüle ergibt sich der weitere Vorteil, dass der mittlere Bereich, der bei dem in Figur 1 gezeigten Ausführungsbeispiel vom Wundkissen 5 eingenommen wird, haftkleberfrei belassen wird, so dass keinerlei Gefahr besteht, dass das Pflaster an der Kanüle o.dgl., die es fixieren soll, zu stark anhaftet, so dass beim Ablösen des Pflasters die Kanüle versehentlich mit herausgezogen wird, oder - umgekehrt - beim Ablösen der Kanüle das Pflaster unkontrolliert mit abgerissen wird.

Es kommen auch wirkstoffhaltige Pflaster (z.B. Rheuma-Pflaster) in Frage, bei denen dann die äußere Klebezone die erforderliche Haftkraft bereitstellt und die innere Klebezone nur so stark haften muss, dass der für die gewünschte Wirkung erforderliche Hautkontakt gegeben ist. Dabei handelt es sich oft um sehr großflächige Pflaster, deren Abziehen in Folge von Hautreizungen oder auch nur als Folge einer Körperbehaarung sehr schmerzhaft sein kann. Erfindungsgemäß werden diese Nachteile erheblich reduziert.

Als Anwendungsgebiet kommen auch die sog. Inzisionsfolien in Frage. Dabei handelt es sich um mit einer Applikationshilfe versehene Folien, die vor einem operativen Eingriff auf die Haut geklebt werden, um ein Auseinanderklaffen eines Einschnitts während der Operation zu verhindern.

Diese Schichten, die die beiden Klebezonen 3 und 4 bilden, können auf bekannte Art aufgebracht werden z.B. durch entsprechend getakteten Sprüh- oder Schlitzkopfauftrag oder durch Sieb- oder Tiefdrucktechniken. Es kann auch verfahrenstechnisch vorteilhaft sein, zunächst die gesamte Unterlage 6 mit einer stark haftenden Schicht zu versehen und dann auf die Fläche für die erste Klebezone 3, begrenzt durch die Linie 7, eine zweite Beschichtung mit einer schwach haftenden Schicht aufzubringen, so dass die stärker haftende Schicht nur in der äußeren Klebezone wirksam wird, während sie in der inneren Klebezone 2 durch die schwächer klebende Schicht abgedeckt ist.

Die Übergänge zwischen den beiden Klebezonen können bezüglich der Haftkräfte fließend, d.h. stufenlos, ausgebildet sein. Unter die Erfindung fallend ist es also auch anzusehen, wenn von der äußeren Kante des Pflasters bis hin zum Wundkissen 5 ein stetiger Übergang, insbesondere mit konstantem Gradienten, von der stärksten bis zur geringsten Haltekraft gegeben ist.

Figur 2 zeigt einen Pflasterstreifen, der sich von dem Pflaster nach Figur 1 dadurch unterscheidet, dass der haftende Bereich 2 den nicht haftenden Bereich, gebildet durch das Wundkissen 5, nicht vollständig, sondern nur quer zur Längsrichtung des Pflasterstreifens umgibt.

Beim Ausführungsbeispiel nach Figur 3 weist die äußere Klebezone 4, die die größere Haftkraft hat, an der Ecke 7 eine kleine die Form eines Streifens 8 aufweisende Lücke auf. Diese Lücke ist mit dem Haftkleber der Klebezone 3 (geringere Haftkraft) beschichtet. Somit ergibt sich ein Streifen 8 der Klebezone 3 zum Rand des Pflasters bzw. dessen Ecke 7 hin. Das dadurch entstehende Eckstück 9 ist als Fingerlift zum Anfassen beim Abziehen des Pflasters besonders geeignet.

Die mehrfach erwähnte Haftkraft wird wie folgt gemessen: man misst die Kraft, die erforderlich ist, um ein Klebeband, z.B. ein Pflaster, von einem planen Untergrund im Winkel von 90° mit konstanter Geschwindigkeit abzuziehen. Dazu wird ein Probestreifen zunächst vier Stunden bei 105° C vorgetrocknet und dann 20 Stunden lang in Standardklima (Raumteperatur 23°C und 50% Luftfeuchte) gelagert. Bei verzugsempfindlichen Produkten wird ein entsprechendes Verstärkungstape verwendet. Unmittelbar vor der Prüfung wird das Probenmaterial mit einer Geschwindigkeit von ca. 30 cm/sek. von der Rolle abgezogen. Dann wird ein Streifen von 400 mm Länge und der vorgegebenen Breite (z.B. 25 mm) geschnitten. Bei Probenstreifen mit rückseitigem Deckpapier erfolgt erst der Zuschnitt, dann die Entfernung des Deckpapiers. Die Probe wird mit der beschichteten Seite in der Mitte dieser Platte aufgetragen, und zwar parallel zur Längskante der Platte. Die Platte wird vorher mit einem benzin- oder butanongetränktem Wattebausch gereinigt. Dann wird in einem geeignetem Behälter Toluol bis zum Sieden erhitzt und die Platte so über den Behälter gehängt, dass sie nicht in direkte Berührung mit der Flüssigkeit kommt. Wenn die Dämpfe den oberen Rand der Platte erreicht haben, wird dieser Zustand fünf Minuten lang aufrechterhalten. Dann wird die Platte herausgenommen; man lässt sie etwa 30 Minuten lang in Standardklima (s.o.) erkalten. Nach dem Auftragen der Probe auf die Platte wird mit einem Finger sanft über die Probe gestrichen, um Lufteinschlüsse zu beseitigen. Anschließend wird die Probe mit einem Tape-Applikator bei 20 N/cm Probenbreite angerollt. Die Platte soll dabei so wenig wie möglich berührt werden, um eine Erwärmung zu vermeiden. Zur Messung wird das Ende des Probestreifens freigelegt und zurückgeschlagen und etwa 25 mm vom Ende der Stahlplatte abgezogen. Die Platte wird in eine Prüfvorrichtung (Zugprüfmaschine nach DIN 51221 Klasse 1) zwischen Auflagefläche und Walzen gelegt, so dass sie leicht zu schieben ist. Das Probenende wird zwischen beiden Walzen geführt und in die obere Klemme der Zugprüfmaschine eingespannt. Der Abzugswinkel beträgt 90°. Die Abzugsgeschwindigkeit wird auf 300 mm pro Minute eingestellt. Nach Zurückstellen der Messwertanzeige auf null wird die Messung durchgeführt. Zur Ermittlung der Klebekraft wird der Kraftverlauf durch Schreiber oder PC aufgezeichnet. Aus den erhaltenen Kraftspitzen wird die mittlere Klebekraft wie folgt ermittelt. Wenn das Diagramm mehr als 20 deutlich erkennbare Kraftspitzen aufweist, dann werden von der Mitte jeder Diagrammlänge, die von der ersten Kraftspitze bis zum Abriss reicht, vier senkrechte Linien in gleichen Abständen von 1/10 der Diagrammlänge nach beiden Seiten eingezeichnet. Die neun Spitzenwerte, die diesen Linien am Nächsten liegen, werden zur Bestimmung der Klebekraft herangezogen. Extrem aus dem Kurvenverlauf herausragende Spitzenwerte werden nicht berücksichtigt. Das Ergebnis wird als Mittelwert von mindestens drei Prüfungen in N/25 mm auf eine Nachkommastelle gerundet ermittelt und angegeben. Die Klebekraft wird wie folgt berechnet:

$$F = \left( \sum_{i=1}^{n} F_i \right) / n$$

wobei $F_i$ die Kraftspitzen $F_1$, $F_2$ ... $F_n$ und n die Anzahl der berücksichtigten Kraftspitzen sind.

**Patentansprüche**

1.  Pflaster, das durch ein flächiges Element gebildet wird, auf dem ein haftender Bereich (2) vorgesehen ist, der bei Draufsicht auf die Applikationsseite einen nicht haftenden Bereich oder ein Wundkissen (5) umgibt, **dadurch gekennzeichnet, dass** der haftende Bereich eine erste Klebezone (3), die den nicht haftenden Bereich oder das Wundkissen (5) umgibt, sowie eine zweite Klebezone (4), die außerhalb der ersten Klebezone (3) angeordnet ist und diese umgibt, umfasst, und dass die Haftkraft der ersten Klebezone (3) mit 0,5-2 N/25mm geringer als die der zweiten Klebezone (4) mit 2-8 N/25mm ist.

2.  Pflasterstreifen, der durch ein streifenförmiges flächiges Element gebildet wird, auf dem ein nicht haftender Bereich oder ein Wundkissen in einer Längsrichtung erstreckt vorgesehen ist und beidseits des nicht haftenden Bereichs oder des Wundkissens ebenfalls in Längsrichtung erstreckte haftende Bereiche (2) vorgesehen sind, welche den nicht haftenden Bereich oder das Wundkissen bei Draufsicht auf die Applikationsseite quer zur Längsrichtung begrenzen, **dadurch gekennzeichnet, dass** die haftenden Bereiche eine erste in Längsrichtung erstreckte innere Klebezone (3), die den nicht haftenden Bereich oder das Wundkissen (5) begrenzt, und eine zweite in Längsrichtung erstreckte äußere Klebezone (4) umfasst, und dass die Haftkraft der ersten Klebezone (3) mit 0,5-2 N/25mm geringer als die der zweiten Klebezone (4) mit 2-8 N/25mm ist.

3. Inzisionspflaster, das durch ein flächiges Element gebildet wird, auf dem ein haftender Bereich (2) vorgesehen ist, **dadurch gekennzeichnet, dass** der haftende Bereich eine erste Klebezone (3) sowie eine zweite Klebezone (4), die außerhalb der ersten Klebezone (3) angeordnet ist, umfasst, und dass die Haftkraft der ersten Klebezone (3) mit 0,5-2 N/25mm geringer als die der zweiten Klebezone (4) mit 2-8 N/25mm ist.

4. Pflaster nach Anspruch 1, 2 oder 3, **dadurch gekennzeichnet, dass** die Unterlage (6) für die Klebezonen (3, 4) durch ein textiles Flächengewebe, z.B. einem Gewebe, Gestrick oder Gewirk, gebildet wird.

5. Pflaster nach Anspruch 1, 2 oder 3, **dadurch gekennzeichnet, dass** die Unterlage (6) für die Klebezonen (3, 4) durch ein Vlies, ein Vlieslaminat oder eine Folie gebildet wird.

6. Pflaster nach einem der Ansprüche 1-5, **dadurch gekennzeichnet, dass** das Pflaster eine Inzisionsfolie oder ein Fixierpflaster ist.

7. Pflaster nach einem der Ansprüche 1-6, **dadurch gekennzeichnet, dass** die Unterlage (6) für die Klebezonen (3, 4) mehrschichtig und/oder als heterogene Struktur ausgebildet ist.

8. Pflaster nach einem oder mehreren der vorstehenden Ansprüche, **dadurch gekennzeichnet, dass** der Übergang von starker Haftkraft zu geringer Haftkraft ein stetiger ist.

9. Pflaster nach Anspruch 8, **dadurch gekennzeichnet, dass** der Übergang mit einem kontinuierlichen, vorzugsweise konstanten Gradienten erfolgt.

10. Pflaster nach einem oder mehreren der vorstehenden Ansprüche, **dadurch gekennzeichnet, dass** der haftende Bereich insgesamt eine erste Klebeschicht umfasst und dass im Bereich der ersten Klebezone (3) oder der zweiten Klebezone (4) eine zweite Klebeschicht auf die erste Klebeschicht aufgebracht ist.

11. Pflaster nach Anspruch 10, **dadurch gekennzeichnet, dass** die erste Klebeschicht diejenige der zweiten Klebezone (4) mit starker Haftkraft und die zweite Klebeschicht diejenige der ersten Klebezone (3) mit geringer Haftkraft ist.

12. Pflaster nach Anspruch 1 oder einem der Ansprüche 3-11, **dadurch gekennzeichnet, dass** sich von der ersten inneren Klebezone (3) mit geringerer Kraft als Fingerlift ein Flächenstück, vorzugsweise ein Streifen (8), mit derselben Haftkraft wie der der ersten Klebezone (3) bis zum Rand des Pflasters, vorzugsweise bis zu einer Ecke (7), hin erstreckt.

**Claims**

1. Plaster which is formed by a plane element, on which an adhesive region (2) is provided, which in plan view of the application side surrounds a non-adhesive region or a wound cushion (5), **characterised in that** the adhesive region comprises a first adhesive zone (3) which surrounds the non-adhesive region or the wound cushion (5), and a second adhesive zone (4) which is arranged outside the first adhesive zone (3) and surrounds it, and **in that** the adhesiveness of the first adhesive zone (3) at 0.5 to 2 N/25 mm is less than that of the second adhesive zone (4) at 2 to 8 N/25 mm.

2. Plaster strip which is formed by a strip-shaped plane element on which a non-adhesive region or a wound cushion extended in a longitudinal direction is provided and adhesive regions (2), also extended in the longitudinal direction, are provided on both sides of the non-adhesive region or of the wound cushion which regions delimit the non-adhesive region or the wound cushion in plan view of the application side transversely to the longitudinal direction, **characterised in that** the adhesive regions comprise a first inner adhesive zone (3) extended in the longitudinal direction and delimiting the non-adhesive region or the wound cushion (5) and comprise a second outer adhesive zone (4) extended in the longitudinal direction, and **in that** the adhesiveness of the first adhesive zone (3) at 0.5 to 2 N/25 mm is less than that of the second adhesive zone (4) at 2 to 8 N/25 mm.

3. Plaster for an incision, which is formed by a plane element on which an adhesive region (2) is provided, **characterised in that** the adhesive region comprises a first adhesive zone (3) and a second adhesive zone (4) which is arranged outside the first adhesive zone (3), and **in that** the adhesiveness of the first adhesive zone (3) at 0.5 to 2 N/25 mm is less than that of the second adhesive zone (4) at 2 to 8 N/25 mm.

4. Plaster according to claim 1, 2 or 3, **characterised in that** the substrate (6) for the adhesive zones (3, 4) is formed by a textile fabric, for example a woven or knitted fabric.

**5.** Plaster according to claim 1, 2 or 3, **characterised in that** the substrate (6) for the adhesive zones (3, 4) is formed by a non-woven fabric, a non-woven laminate or a film.

**6.** Plaster according to one of claims 1 to 5, **characterised in that** the plaster is an incision film or a fixing plaster.

**7.** Plaster according to one of claims 1 to 6, **characterised in that** the substrate (6) for the adhesive zones (3, 4) is designed so as to be multilayered and/or as a heterogeneous structure.

**8.** Plaster according to one or more of the preceding claims, **characterised in that** the transition from strong adhesiveness to slight adhesiveness is a constant one.

**9.** Plaster according to claim 8, **characterised in that** the transition takes place with a continuous, preferably constant, gradient.

**10.** Plaster according to one or more of the preceding claims, **characterised in that** the adhesive region as a whole comprises a first adhesive layer, and **in that** a second adhesive layer is applied to the first adhesive layer in the region of the first adhesive zone (3) or the second adhesive zone (4).

**11.** Plaster according to claim 10, **characterised in that** the first adhesive layer is that of the second adhesive zone (4) with strong adhesiveness and the second adhesive layer is that of the first adhesive zone (3) with slight adhesiveness.

**12.** Plaster according to claim 1 or one of claims 3 to 11, **characterised in that** a flat piece, preferably a strip (8) with the same adhesiveness as the first adhesive zone (3), extends from the first inner adhesive zone (3) with less force than the lifting of a finger to the edge of the plaster, preferably to a corner (7).

**Revendications**

**1.** Pansement adhésif, qui est formé par un élément plat, sur lequel est prévue une zone adhésive (2) qui, sur une vue de dessus de la face d'application, entoure une zone non adhésive ou une compresse (5), **caractérisé en ce que** la zone adhésive comporte une première zone de collage (3), qui entoure la zone non adhésive ou la compresse (5), ainsi qu'une deuxième zone de collage (4), qui est disposée en dehors de la première zone de collage (3) et entoure celle-ci, et **en ce que** la force adhésive de la première zone de collage (3), avec une valeur de 0,5 à 2 N/25 mm, est inférieure à celle de la deuxième zone de collage (4) avec une valeur de 2 à 8 N/25 mm.

**2.** Bande de pansement adhésif, qui est formée par un élément plat en forme de bande, sur lequel est prévue une zone non adhésive ou une compresse s'étendant dans le sens longitudinal, et sur lequel sont prévues des zones adhésives (2) qui s'étendent également dans le sens longitudinal de part et d'autre de la zone non adhésive ou de la compresse, lesquelles délimitent la zone non adhésive ou la compresse transversalement par rapport au sens longitudinal sur une vue de dessus de la face d'application, **caractérisée en ce que** les zones adhésives comportent une première zone de collage intérieure (3), qui s'étend dans le sens longitudinal et qui délimite la zone non adhésive ou la compresse (5), et une deuxième zone de collage extérieure (4) qui s'étend dans le sens longitudinal, et **en ce que** la force adhésive de la première zone de collage (3), avec une valeur de 0,5 à 2 N/25 mm, est inférieure à celle de la deuxième zone de collage (4) avec une valeur de 2 à 8 N/25 mm.

**3.** Pansement adhésif pour incisions, qui est formé par un élément plat, sur lequel est prévue une zone adhésive (2), **caractérisé en ce que** la zone adhésive comporte une première zone de collage (3), ainsi qu'une deuxième zone de collage (4), qui est disposée en dehors de la première zone de collage (3), et **en ce que** la force adhésive de la première zone de collage (3), avec une valeur de 0,5 à 2 N/ 25 mm, est inférieure à celle de la deuxième zone de collage (4) avec une valeur de 2 à 8 N/25 mm.

**4.** Pansement adhésif selon la revendication 1, 2 ou 3, **caractérisé en ce que** le support (6) des zones de collage (3, 4) est formé par une structure plane textile, telle qu'un tissu, un tricotage ou un maillage.

**5.** Pansement adhésif selon la revendication 1, 2 ou 3, **caractérisé en ce que** le support (6) des zones de collage (3, 4) est formé par un non tissé, un non tissé stratifié ou un film plastique.

**6.** Pansement adhésif selon l'une quelconque des revendications 1 à 5, **caractérisé en ce que** le pansement adhésif est un pansement pour incisions en film plastique ou un pansement de fixation.

**7.** Pansement adhésif selon l'une quelconque des revendications 1 à 6, **caractérisé en ce que** le support (6) des zones de collage (3, 4) est conçu sous forme de structure multicouche et/ou de structure hétérogène.

**8.** Pansement adhésif selon l'une ou plusieurs des revendications précédentes, **caractérisé en ce que** la transition entre la force adhésive élevée et la force adhésive faible est une transition continue.

**9.** Pansement adhésif selon la revendication 8, **caractérisé en ce que** la transition est effectuée avec un gradient continu, de préférence constant.

**10.** Pansement adhésif selon l'une ou plusieurs des revendications précédentes, **caractérisé en ce que** la zone adhésive comprend sur toute sa surface une première couche de colle et **en ce qu'**une deuxième couche de colle est appliquée sur la première couche de colle dans la zone de la première zone de collage (3) ou de la deuxième zone de collage (4).

**11.** Pansement adhésif selon la revendication 10, **caractérisé en ce que** la première couche de colle est celle appliquée sur la deuxième zone de collage (4) avec la force adhésive élevée et la deuxième couche de colle est celle appliquée sur la première zone de collage (3) avec la force adhésive plus faible.

**12.** Pansement adhésif selon la revendication 1 ou l'une quelconque des revendications 3 à 11, **caractérisé en ce qu'**une partie plane, de préférence une bandelette (8), formant une zone de prise pour les doigts et présentant la même force adhésive que la première zone de collage (3), s'étend à partir de la première zone de collage intérieure (3), avec la force adhésive plus faible, jusque sur le bord du pansement, de préférence jusqu'à un coin (7).

Fig. 1

Fig. 2

Fig. 3